# EUROPEAN PATENT SPECIFICATION

(11) **EP 0 470 660 B1**
(45) Date of publication and mention of the grant of the patent: **19.07.1995**
(21) Application number: 91201948.6
(22) Date of filing: 25.07.1991
(51) Int. Cl.: A61B 17/60

(54) **Apparatus for the correction of scoliosis**
Vorrichtung zum Korrigieren von Skoliose
Dispositif pour la correction de la scoliose

(30) Priority: 07.08.1990 NL 9001778
(43) Date of publication of application: 12.02.1992
(73) Proprietor: ACROMED B.V., NL-3003 AD Rotterdam (NL)
(72) Inventor: Cool, Jan Constant, NL-2641 AC Pijnacker (NL); Sanders, Marcus Maria, NL-7511 CJ Enschede (NL); Veldhuizen, Albert Gerrit, NL-9761 HE Eelde (NL)
(74) Representative: Flamman, Han

(56) References cited:
- EP-A- 0 140 790
- EP-A- 0 330 881
- FR-A- 2 624 720
- US-A- 3 786 806
- US-A- 4 289 123
- Article "Medici passen metaallegering toe" De Ingenieur/ JRG 90/ NR. 25 Juni 1978

## Description

The invention relates to a rod with elastic properties for correcting the shape of a spinal column, whereby the rod is attached to the vertebrae of the spinal column which has to be corrected as to bends and possibly twists in the longitudinal direction thereof, at the rear side thereof, across the part which is to be corrected and is bent and possibly twisted, corresponding with the curve and possibly the torsion of the spinal column.

An important part of orthopaedics is the treatment of patients with a serious shape deformation (scoliosis) of the spinal column and the chest. The shape deformations referred to here are: lateral curvature and axial twisting.

Especially if these shape deformations amount to more than 30°, they can lead to a reduction of the lung function and a reduced life expectancy. Light to medium deformations of this type occur in 0.2 to 0.3% of the 16 year olds, and 10 times as much in women than in men.

Lateral curves of 45° (the curves are measured in co-called Cobb angles: the angle between the normals at the ends of the curved part of the spinal column viewed from the rear) or more are qualified as "serious deformations" and are generally treated surgically.

A known, much used surgical method is the so-called Harrington method: hooks are placed under the arches of the vertebrae which mark the end of the curved/deformed part, with which a stainless steel rod is held in place. The part of the rod which is situated between the hooks is then "stretched", so that the hooks and the corresponding end-vertebrae are pushed away from each other: - the "curvature" thus is decreased. As a result of the visco-elastic qualities of the spinal column this can, after the initial stretching, be stretched further for some time more - for example 10 minutes. The degree to which stretching is possible is determined by the forces which the spinal column can bear without damage arising. After this treatment. bone is taken from the pelvis and laid next to the rod against vertebrae of the spinal columns stretched by the rod. In 3 or 4 months this leads to so-called "fusion" (growing together and thereby stiffening) of the vertebrae concerned. When this "fusion" is a fact the rod is generally removed again.

It turns out that with the aid of this method a reduction of the Cobb angle of approximately 50% can be achieved. As far as the correction of the torsions is concerned this known method has no possibilities.

In the published European patent application no. 84.402149.3 (publication no 0140790) a rod according to the preamble of claim 1 is disclosed. In this document a description is given as to how a deformation of the spinal column can he corrected by attaching a construction to the spinal column, in the longitudinal direction thereof, which contains elastic material. The construction described in the said patent application consists of a bundle of parallel elastic rods. This construction is bent and/or twisted, corresponding to the shape of the spinal column at the location which is to be corrected. The construction will want to become "straight" again and will exert a force on the spinal column via the vertebrae to which it is attached. As a result of the visco-elastic qualities of the spinal column it can be corrected under the influence of the said force. The desired result is therefore achieved by exposing the spinal column during a longer period of time to a - stretching - force.

In the said patent application a description is given as to how, when using such a construction: the size of the force exerted on the spinal column is dependent on the degree of the deformation. Therefore, as the deformation decreases - during the course of time - the correcting force decreases.

In practice it appears that the best results when correcting deformations are reached if the spinal column is not exposed to a force which gradually decreases with decreasing deformation, but to a constant force. This especially is required in the case that the deformation is a curvature coupled with a torsion. Surprisingly, it has now turned out that such a constant force can be achieved by making use of a rod of a material which has a shape memory. Shape memory means, that the material, when deformed below a certain temperature - the so-called transition temperature - and then heated above that temperature "remembers" its original shape and wants to resume that shape. If it is obstructed in resuming that shape, it will exert a force on the obstructing element. This resuming force is known to decrease with the decreasing deformation: the shape-memory-behaviour. However: with a degree of deformation above a certain value: which value is specific for the shape-memory-alloy used, over a long deformation-range the resuming force remains fairly constant. This area of deformation is called the area of pseudo-plastic behaviour.

The object of the invention is to provide rods to be used for the afore mentioned purpose, exerting the desired constant force on the spinal column. As stated herein before: shape-memory-alloys do provide such a constant force, however only when deformed above a certain degree.

The rod according to the invention to that end is characterized in that the rod consists of a material with a shape memory with an appertaining transition temperature, which is lower than the body temperature, with a substantial range of deformation with pseudo-plastic behaviour and with a cross-section with linear dimensions, in case of a bent rod in the direction of the bending and in case of a twisted rod radially outwards from the center, such that in use, above the transition temperature, it shows a pseudo-plastic behaviour over a range, sufficiently long to exert a constant force throughout the correcting process.

The attachment to the vertebrae is usually done to alternative vertebrae, which means: per movement segment (vertebra - intervertebral disc - vertebra). This is done because it is often difficult due to lack of space to attach an attachment element to each vertebra.

Each spinal column which is to be corrected will set its own specific requirements with regard to the size of the force which can, or respectively must, be applied. The size of that force - and also the transition temperature - can be set by making a correct choice as concerns the composition and the sizes of the rod which is to be used. Rods of various compositions and sizes therefore need to be available to a surgeon. It has appeared, that Ti-Ni alloys suffice especially well as material with a shape memory for the aims which have been set: preferably in the atomic ratio of about 1:1. The forces which arise are, across a long correction trajectory - for example: 8% stretch - constant to a good degree; the size of the force can be set with the aid of changes in the Ti-Ni ratio. A further advantage of the Ti-Ni alloys is that they are relatively easily shapeable at room temperature or at a lower temperature. A rod thereof can therefore be easily bent and/or twisted in such a manner that it "fits" with the curve of the part of a spinal column which is to be corrected and can be attached to vertebrae of that part.

Experiments have furthermore shown that in particular with linear measurements in the perpendicular cross-section of the rod of about 5 mm, the constant force which is sought can always be achieved. In order to exactly achieve a certain shape of a part of a spinal column, such as the natural S-shape of the spinal column from a lateral view, it is advantageous to pre-program a rod before it is attached to the vertebrae with the aid of attachment elements, for example with the aid of a memory heating. In the case of "the natural S-shape" it is the case, however, that a certain S-shape will arise also without pre-shaping, namely if the effect of the force is small with slight curves of the memory metal. The time necessary for a correction is, even for large deformations of for example 100°, such - three to four weeks - that the correction has been completed when the vertebrae begin to fuse - see with regard to "fusion" the Harrington method mentioned above. May it be noted, that it is, however, improbable that such large angles will still be found at the present time. In general corrections are made before such large deformations arise. A disadvantage of an especially large curve of for example 100° is that the growing together appurtaining thereby is also very serious. Whether the visco-elastic behaviour is also then sufficient for full correction within 4 weeks is the question. The size of the force which arises can be increased by using a rod of a material with a low transition temperature, for example lower than room temperature. The staff is then inserted undercooled instead of at room temperature.

In particular it is advised to use rods with a rectangular cross-section, advantages being that they are easy to deform as they offer an easy grip for pliers and that they are easy to rigidly secure in the attachment elements.

A large advantage of using a rod according to the invention is therefore that it can be adjusted entirely to the specific requirements which are laid down in a certain case: by means of a suitable choice of the material composition and the sizes of the rod which is to be used.

The invention also contains the combination of rod and attachment elements - a set -, as is applied for the implementation of the said treatment. Rods and attachment elements are adapted to one another and in such a manner that the rod has a cross-section with which it fits in a translation free and rotation free manner into grooves corresponding therewith running in the longitudinal direction of the attachment elements.

In order to prevent the rod from moving in the attachment element the latter is equipped with means to anchor the rod therein. For example, attachment is achieved with the aid of a snap lock. Use is also sometimes made of "covers" on top of the groove, in order to thus confine the rod. Or use is also made of screws.

In a preferable design of an attachment element according to the invention this contains an arm which extends approximately perpendicularly to the axial direction of the spinal column around the vertebra and is attached at its other end to the vertebra. The said arm especially fulfills an important function when transferring torsion forces to the vertebrae.

May it be noted, that a small corrective force continues to work on the vertebrae also in the resting position of the rod. When the spinal column has the inclination to curve back again when the rod is present, this force increases. A rod according to the invention can thus be used as an internal corset: it is then no longer necessary to allow the vertebrae to fuse.

The invention will be explained further with the aid of a few figures and with the aid of the drawing.

Experiments have been carried out on spinal columns with lateral curves of 60° to 100° Cobb. The twisting which occurred amounted to between 40° to 45°. Twisting is here taken to mean: the twisting of the most twisted vertebra - approximately the middle vertebra of a deformed part - with regard to a non- twisted - the last - vertebra of a deformed part.

A rod of a Ti-Ni alloy was used, with an atomic ratio of 1:1, with a square cross-section of 5x5 mm. The rod was bent and twisted at room temperature and was attached into the attachment elements with the aid of a pin which was inserted with a pair of pliers between the rod and the wall of the container. The rod was closed-in in the container with the aid of a cover. It always turned out that within 4 weeks - the time after which the vertebrae start to grow into one another - the final position of the spinal column was reached.

The lateral force was in the order of size of 150 N; 5 Nm was used as a maximum for the moment of torsion.

After being applied the rod was heated to 42 °C, after which it cooled to room temperature. The force which arose did not decrease thereby.

The same kind of experiments have been carried out with a rod of a rectangular cross-section of 4x7 mm. The rod was bent and twisted at room temperature and was anchored in the attachment elements with the aid of a snap lock. The 7 mm side of the cross-section was situated in the medio-lateral direction - i.e.: in the plane in which the sideward curve is situated. Also here it appeared that within four weeks - the time needed for vertebra to start to grow together - the utmost position of the spinal column was reached. However, during that four weeks the correction was realised much more gradually and controlled than in the example described hereinbefore, because of the better pseudo-elastic action of the rod as a result of the larger width of the rod. Thus it was possible to realise an increased correction.

In the drawing the figures 1 and 2 are rear views of a spinal column, figure 3 shows a rod according to the invention and figure 4 shows an example of an appurtaining attachment element.

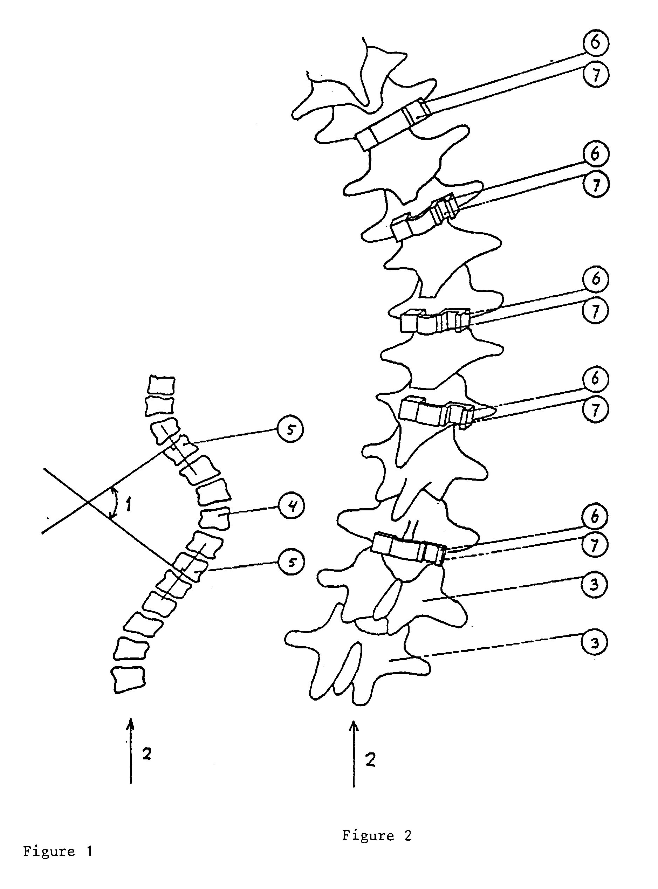

In Fig. 1, 1 shows the lateral curvature of the spinal column 2 - the so-called Cobb angle. The spinal column 2 consists of a piling-up of vertebrae 3. The vertebra 4, situated in approximately the middle of the curved - and therefore to be corrected - part of the spinal column will be twisted to the greatest degree. The twisting is determined by the position of vertebra 4 in comparison with that of the end vertebrae 5 and the part which is to be corrected.

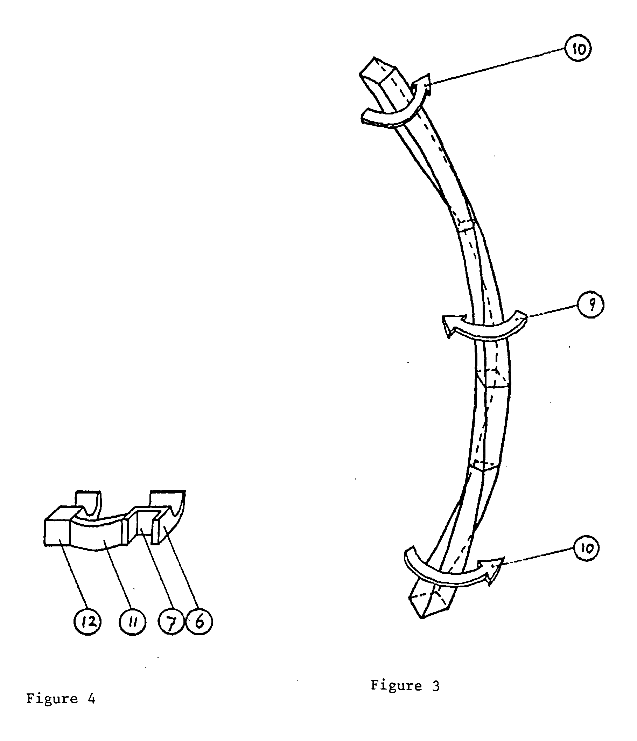

The spinal column 2, shown in Fig. 2, also shows a rear view. It shows how the attachment elements have grooves 7, in which the rod which is to be applied fits. That rod 8 is shown in fig. 3. It is a rod with a square cross-section, consisting of a Ti-Ni alloy. Before it is laid into the groove 7 of the attachment element 6 it is bent and twisted - see the arrows 9 and 10 in Fig. 3 - in accordance with the form of the spinal column 2 which is to be corrected.

Fig. 4 shows an implementation example in detail of an attachment element 6. The groove is shown by 7, in which the rod 8 fits. The element 6 has a - short - arm part 11, which grips around an extension of the vertebra 3 with the aid of a hook 12, thus fixing it.

## Claims

1. Rod with elastic properties for correcting the shape of a spinal column (2), whereby the rod is attached to the vertebrae (3) of the spinal column (2) which has to be corrected as to bends (9) and possibly twists (10) in the longitudinal direction thereof, at the rear side across the part to be corrected and is bent and possibly twisted, corresponding to the curve and possible torsion of the spinal column, characterized in that the rod consists of a material with a shape memory with an appertaining transition temperature, which is lower than the body temperature, with a substantial range of deformation with pseudo-plastic behaviour and with a cross-section with linear dimensions in the direction of the bending and, in case of a twisted rod, radially outwards from the center, such that in use, above the transition temperature, it shows a pseudo-plastic behaviour over a range, sufficiently long to exert a constant force throughout the correcting process.

2. Rod according to claims 1,
characterized in that the rod consists of a alloy of Ti and Ni in the atomic ratio of approximately 1:1.

3. Rod according to the claims 1 or 2,
characterized in that the linear size in the cross-section of the rod is about 5 mm.

4. Rod according to one of the preceding claims,
characterized in that it has a rectangular cross-section.

5. Set containing one or more rods according to claim 1-4 and containing attachment elements (6), in which the rods fit in a translation-free and rotation-free manner in corresponding grooves (7) running in the longitudinal direction of the elements (6), the grooves (7) having cross-sections corresponding to that of the rods.

6. Set containing one or more rods according to the claims 1-4,
characterized in that the attachment elements (6) are equipped with a snap lock to fix a rod therein.

7. Set according to claim 5,
characterized in that the attachment elements (6) are equipped with small covers, to lock a rod into a groove.

8. Set according to one of the claims 5-7,
characterized in that the attachment elements (6) contain arms (11) which extend approximately perpendicularly to the axial direction of the spinal column around a vertebra (3) and are attached to the vertebra at their other end.

## Patentansprüche

1. Stab mit elastischen Eigenschaften zum Korrigieren der Form einer Wirbelsäule (2), wobei der Stab an den Wirbeln (3) der Wirbelsäule (2) befestigt wird, die hinsichtlich Krümmungen (9) und möglicherweise Torsionen (10) in ihrer Längsrichtung auf ihrer Rückseite korrigiert werden soll, quer zu dem Teil, das korrigiert werden soll, und entsprechend der Krümmung und möglichen Torsion der Wirbelsäule gebogen und möglicherweise verdreht wird,
dadurch gekennzeichnet, daß der Stab aus einem Werkstoff mit einem Formgedächtnis besteht, mit einer zugehörigen Umwandlungstemperatur, die niedriger als die Körpertemperatur ist, mit einem bedeutenden Bereich an Verformung mit pseudo-plastischem Verhalten und mit einem Querschnitt mit Längenabmessungen in der Richtung der Krümmung und im Fall eines verdrehten Stabes vom Mittelpunkt radial nach außen, auf eine Weise, daß er in der Anwendung oberhalb der Umwandlungstemperatur ein pseudo-plastisches Verhalten über einen Zeitraum zeigt, der genügend lang ist, um während des gesamten Korrektur-Vorgangs eine konstante Kraft auszuüben.

2. Stab gemäß Anspruch 1,
dadurch gekennzeichnet, daß der Stab aus einer Legierung aus Ti und Ni mit einem Atomverhältnis von ungefähr 1:1 besteht.

3. Stab gemäß Anspruch 1 oder 2,
dadurch gekennzeichnet, daß die Abmessung des Querschnitts des Stabes ungefähr 5 mm ist.

4. Stab gemäß einem der vorhergehenden Ansprüchen,
dadurch gekennzeichnet, daß er einen rechteckigen Querschnitt hat.

5. Set, das einen oder mehrere Stäbe gemäß Anspruch 1-4 enthält und das Befestigungs-Elemente (6) enthält, in die die Stäbe auf translations-freie und rotations-freie Weise in entsprechende Aussparungen (7) paßt, die in Längsrichtung der Elemente (6) verlaufen, wobei die Aussparungen (7) einen Querschnitt haben, der dem der Stäbe entspricht.

6. Set, das einen oder mehrere Stäbe gemäß Anspruch 1-4 enthält,
dadurch gekennzeichnet, daß die Befestigungs-Elemente (6) mit einer Einrast-Vorrichtung ausgerüstet sind, um einen Stab darin zu befestigen.

7. Set gemäß Anspruch 5,
dadurch gekennzeichnet, daß die Befestigungs-Elemente (6) mit kleinen Deckeln versehen sind, um einen Stab in einer Aussparung zu verschließen.

8. Set gemäß einem der Ansprüche 5-7,
dadurch gekennzeichnet, daß die Befestigungs-Elemente (6) Arme (11) besitzen, die sich ungefähr senkrecht zur axialen Richtung der Wirbelsäule um einen Wirbel (3) erstrecken und mit ihrem anderen Ende an dem Wirbel befestigt sind.

## Revendications

1. Une tige avec des propriétés élastiques pour corriger la forme d'une colonne vertébrale (2), la tige étant attachée aux vertèbres (3) de la colonne vertébrale (2) qui doit être corrigée quant aux courbes (9) et peut-être aux torsions (10) dans sa direction longitudinale, à l'arrière de celle-ci, à travers la partie qui doit être corrigée, et étant courbée et peut-être tordue, suivant la courbe et peut-être la torsion de la colonne vertébrale,
caractérisée en ce que la tige consiste en un matériau avec une mémoire de la forme, avec une température de transition propre qui est inférieure à la température du corps, avec une étendue de déformation substantielle avec un comportement pseudo-plastique, et avec une coupe transversale avec des dimensions linéaires dans le sens de la courbe et, dans le cas d'une tige tordue, radialement vers l'extérieur à partir du centre, de sorte que lors de l'utilisation, au-delà de la température de transition, elle montre un comportement pseudo-plastique sur une étendue suffisamment longue pour exercer une force constante durant le processus de correction.

2. Une tige d'après la revendication 1,
caractérisée en ce que la tige consiste en un alliage de Ti et de Ni, dans le un ratio atomique d'environ 1:1.

3. Une tige d'après la revendication 1 ou 2,
caractérisée en ce que la taille linéaire de la coupe transversale de la tige est d'environ 5 mm.

4. Une tige d'après l'une des revendications précédentes,
caractérisée en ce qu'elle a une coupe transversale rectangulaire.

5. Un dispositif comprenant une ou plusieurs tiges d'après les revendications 1 à 4 et comprenant des éléments de fixation (6), dans lesquels les tiges s'adaptent de façon à pouvoir effectuer des rotations et des translations librement dans des rainures correspondantes (7), situées dans la direction longitudinale des éléments de fixation (6), les rainures (7) possédant des coupes transversales correspondant à celles des tiges.

6. Un dispositif comprenant une ou plusieurs tiges d'après les revendications 1 à 4,
caractérisé en ce que les éléments de fixation (6) sont équipés d'un verrou rapide afin d'y fixer une tige.

7. Un dispositif d'après la rendication 5,
caractérisé en ce que les éléments de fixation (6) sont équipés de petits couvercles, afin d'enfermer une tige dans une rainure.

8. Un dispositif d'après l'une des revendications 5 à 7,
caractérisé en ce que les éléments de fixation (6) contiennent des bras (11), qui s'étendent approximativement perpendiculairement à la direction axiale de la colonne vertébrale autour d'une vertèbre (3), et sont attachés à leur autre extrémité à la vertèbre.
